# EUROPEAN PATENT SPECIFICATION

(11) **EP 2 939 662 B1**
(45) Date of publication and mention of the grant of the patent: **02.10.2019**
(21) Application number: 13869093.8
(22) Date of filing: 20.12.2013
(51) Int. Cl.: A61K 9/16, A61K 31/14, A61K 47/30

(54) **PHARMACEUTICAL COMPOSITION COMPRISING TEMOZOLOMIDE WITH IMPROVED STABILITY AND PROCESS FOR MANUFACTURING THE SAME**
TEMOZOLOMIDHALTIGE PHARMAZEUTISCHE ZUSAMMENSETZUNG MIT VERBESSERTER STABILITÄT UND HERSTELLUNGSVERFAHREN DAFÜR
COMPOSITION PHARMACEUTIQUE AYANT UNE STABILITÉ AMÉLIORÉE, CONTENANT DU TÉMOZOLOMIDE ET SON PROCÉDÉ DE PRÉPARATION

(30) Priority: 31.12.2012 KR 20120158569
(43) Date of publication of application: 04.11.2015
(73) Proprietor: Samyang Biopharmaceuticals Corporation, Seoul 110-725 (KR)
(72) Inventor: LIM, Hye-Jung, Daejeon 305-722 (KR); PARK, Sang-Yeob, Daejeon 305-761 (KR); KIM, Kyung-Hee, Daejeon 302-739 (KR)
(74) Representative: van Kooij, Adriaan
(86) International application number: PCT/KR2013/011973
(87) International publication number: WO 2014/104671

(56) References cited:
- WO-A2-2011/036676
- KR-A- 20120 121 944
- US-A- 5 939 098
- US-A1- 2002 172 662
- US-A1- 2007 112 053
- US-A1- 2008 044 457

## Description

### Technical Field of the Invention

The present invention relates to a granule comprising temozolomide and a method for preparing the same.

### Background of the Invention

Temozolomide(TMZ) is one of the anti-cancer drug approved for the chemotherapy of brain tumor (brand name of Temodar® in US, Temodal® in Europe , commercialized by Schering Corp.). The chemical name of the temozolomide is 3,4-dihydro-3-methyl-4-oxoimidazo[5,1-d]-as-tetrazine-8-carboxamide (US Patent No.5,260,291) and its cytotoxicity is thought to be due to DNA alkylation caused by 5-(3-methyltriazen-1-yl)-imidazole-4-carboxamide (MTIC) which is a metabolite of temozolomide.

US 2002/172662 discloses discloses a method for treating advanced cancer in patients in need of such treating wherein temozolomide and alpha interferon are administered in combination in amounts sufficient to achieve a clinical response.

Temozolomide is used for the treatment of adult patient with newly diagnosed glioblastoma multiforme (GBM) and refractory anaplastic astrocytoma and is approved for the treatment of patients with malignant glioma such as glioblastoma multiforme or anaplastic astrocytoma, when the tumor has recurred or become worse after standard treatment.

Although temozolomide is water-insoluble, it degrades by wet condition, high pH, a high temperature of over a room temperature and light, thereby changing its property of "white to light tan or light pink powder" to "dark pink or tan powder.

Accordingly, in order to prepare a stable formulation of temozolomide, it is necessary to improve its stability.

Also, a capsule formulation of temozolomide is on the market in various dosages such as 5, 20, 100 and 250mg. While the main component is contained at a high dosage per capsule, it is difficult to achieve the full and uniform content, due to the bulky volume and low flowability of drug.

### Detailed Description of the Invention

In order to solve the problem described above, the object of the present invention is to provide granules containing temozolomide and a method for preparing the same with high moisture stability and storage stability by inhibiting related substance formation, while maintaining the dissolution pattern of a conventional formulation including temozolomide.

To achieve the object, the inventors studied and found that the granule-form of temozolomide and a pH stabilizer had improved stability to pH and water, capsule-filling ability and uniformity of content, and the effect was further maximized by forming the granules by a dry-granulation method. In addition, a formulation with excellent properties can be provided by optimizing the components, the contents and the addition time of the pharmaceutically acceptable additives to the dry granules.

According to the present invention there is provided a dry granule containing temozolomide as an active ingredient and tartaric acid as a pH stabilizer.

Another preferred embodiment of the present invention provides a pharmaceutical composition with an improved stability, including the granules and pharmaceutically acceptable additives.

A further embodiment of the present invention provides a method for preparing a pharmaceutical composition with an improved stability comprising: a) mixing temozolomide and tartaric acid as a pH stabilizer; b) forming dry granules from the mixture and sizing the granules; and c) mixing the sized granules with one or more pharmaceutically acceptable additives.

The present invention is described in detail as follows.

The granules of the present invention are dry granules including temozolomide and tartaric acid as a pH stabilizer.

The pH stabilizer controls pH of the granules and the pharmaceutical composition comprising the granules. Tartaric acid is used as a pH stabilizer. It is possible to adjust pH of the pharmaceutical composition to 5 or less, for example pH 2 to 5 by using the pH stabilizer

Preferably, the pH stabilizer is contained at an amount of 1 to 50 parts by weight, or more preferably 3 to 30 parts by weight per 100 parts by weight of the temozolomide.

The granules further comprise an anti-caking agent, preferably.

The anti-caking agent prevents adhesion of hygroscopic ingredients adhering, and can be one or more selected from the group consisting of D-mannitol, colloidal silicon dioxide, calcium silicate, magnesium silicate, and calcium stearic acid, but is not limited thereto. More preferably, the anti-caking agent is a colloidal silicon dioxide that improves granular flowability and compression-moldability by its small particle size and large surface area.

Also, the anti-caking agent may be contained at an amount of 0.05 to 0.1 parts by weight per 100 parts by weight of temozolomide. While an excessive amount of the anti-caking agent may improve the lubricancy, it might affect dissolution or properties of the drug. Thus, it is preferable to use the anti-caking agent in the range, thereby accomplishing the anti-caking property at a minimal amount.

Further, the granule may further include one or more pharmaceutically acceptable additives to improve the workability, flowability and properties of the granule.

All additives except for a main drug as an active ingredient are commonly named as additives or excipients, and may preferably further comprise one or more of excipients, binders, disintegrants, glossing agents, diluents, and combinations thereof in the granule.

The excipients may provide a given volume, good tableting characteristics and uniform content, depending on the amount of the temozolomide. Examples of the excipients may include lactose, mannitol, starch, powdered white sugar, calcium phosphate, microcrystalline cellulose or colloidal silicon dioxide etc. A person skilled in the art may properly choose in light of its cost or conformity with the active ingredient. Other kind of additives may function as an excipient, as well as its main use.

However, when highly hydrophilic additives such as lactose are used in the pharmaceutical composition, they may be formulated by mixing with the prepared granules after granulation process instead of granulating with temozolomide, so as to obtain the preferred dissolution property.

The binders improve the adhesive property of granules and maintain the shape of the compressed formulation. The examples of binders are maltose, natural gum (Gum Arabic), cellulose derivatives (methyl cellulose, carboxymethylcellulose, microcrystalline cellulose), gelatin, povidone and the like, but not limited thereto.

The disintegrants accelerates release or disintegration of formulations, specifically disintegrate tablets in gastrointestinal tract by swelling in contact with moisture. For example, starch derivatives (corn starch, potato starch, sodium starch glycolate, croscarmellose), cellulose derivatives (sodium carboxymethyl cellulose, polyvinyl pyrrolidone), crospovidone, cation exchange resin etc. Preferably, sodium starch glycolate having rapid water absorption and swelling may be used as a disintegrant.

The lubricant improves flowability, prevents granules from adhering to punches or rollers, and decreases outlet-resistance of granules in granule preparation. After granule preparation, it functions to facilitate the filling of the formulations, such as capsules with the prepared granule mixture. Examples of lubricants are talc, hydrogenated castor oil, magnesium stearate, calcium stearate, silicon dioxide, stearic acid, magnesium stearic acid, or mixture thereof, but are not limited thereto.

Additionally, the diluents such as microcrystalline cellulose, lactose, glucose, mannitol, alginate, alkaline-earth metal salts, clay, polyethylene glycol and dicalcium phosphate, and conventionally used desiccants, sweetening agent or moisture absorbent may be used without impairing the effects of the present invention. The preferable types and contents of additives can be selected by a person skilled in the art, in light of component property, dosages and formulation characteristics.

One preferable example of the present invention improves moisturization and uniformity by granulating the temozolomide with pH stabilizer, compared to the mixture prepared by simple-mixing process.

It is difficult to assure uniform stability by inhibiting separation of pH adjusting agent from the mixture in mixing and filling process, while adding tartaric acid as a pH adjusting agent in a low ratio compared to the main drug. The tartaric acid as a pH adjusting agent is transparent crystalline form unlike the bulky temozolomide.

To solve the problem, a wet-granulation method and a dry-granulation method may be applied, using tartaric acid as a pH stabilizer. In the wet-granulation method, crystalline tartaric acid is dissolved in water or organic solvent and then is added to the mixture as a binder, which is a general method in the pharmaceutical field. The wet-granulation method may lower the stability of the temozolomide due to the low stability in water, and changes the drug property by using the organic solvent.

Accordingly, granules according to one preferable embodiment are dry-granules prepared by dry-granulation method.

The term, "Dry granule" used herein refers to the granules formed without using a granulating solvent such as water or ethanol. The term, "wet-granule" refers to the granules formed by using a solvent as a binder and drying aggregated wet-mass in a convection oven or a fluid bed dryer in a proper temperature and time followed by sizing.

In the dry-granulation process, it is economically beneficial, because it is simple and is completed in a short time. Further, it solves the problem of a rapid increase in related substances formed by the wet-type process, and remarkably increases the stability and the drug compliance by maintaining stability even at high temperature and humidity in the summer time. The dry-granulation method is more suitable than the wet-granulation method for minimizing the undesired effect on the stability while maintaining the same properties as the conventional formulation (Experimental Examples 1 to 4).

In the dry-granulation method, the formulation can be obtained by using a roller compacting method or a slugging method, and preferably through roller compacting. Specifically, the roller compacting method prepares granules by compressing a powder under constant pressure, while passing the powder through a gap of two rollers. The roller-compacted mixture may be optionally further sized or sieved with a fitz-mill or an oscillator to obtain proper size of granules, optionally.

One example of the present invention uses the granules together with one or more pharmaceutically acceptable additives to provide a pharmaceutical formulation.

Accordingly, another preferable example of the present invention provides a pharmaceutical formulation with improved stability, comprising the granules and one or more pharmaceutically acceptable additives.

The pharmaceutically acceptable additives are contained in the pharmaceutical formulation by additionally mixing then with the granules, and can be called as a "post-mixed additive" hereinafter, to be distinguished from the additives included inside the granules as described above.

Examples of post-mixed additives may include excipients, stabilizers, lubricants, buffers, sweetening agents, base surfactants, absorbents, flavor enhancers, binders, suspending agents, hardeners, antioxidants, polishes, fragrance ingredients, flavor agents, pigments, coating agents, wet-controlling agents, fillers, antifoaming agents, cooling agents, masticating agents, anti-static agents, colorings, dispersants, disintegrants, waterproof agents, antiseptics, preservatives, solubilizing agents, plasticizers, etc., and can include the additives inside the granules described above, but are not limited thereto. An ordinarily-skilled person can select suitable additives, as well as the described additives.

More preferably, the pharmaceutically acceptable additives (post-mixed additives) may include a mixture of hydrophilic lubricants and hydrophobic lubricants.

The hydrophilic lubricants may be one or more selected from the group consisting of stearic acid, sodium lauryl sulfate and polyethylene glycol (for example, polyethylene glycol having weight-average molecular weight of 4000 or higher) and the hydrophobic lubricants may be one or more selected from the group consisting of magnesium stearic acid, talc, silicon dioxide and starch.

Preferably, the mixture of hydrophilic lubricants and hydrophobic lubricants may include hydrophilic lubricants and hydrophobic lubricants in a weight ratio of 2:1 to 1:5. By using the lubricant mixture in the ratio, provided is the composition containing temozolomide which has improved properties such as good flowability and high stability, while maintaining dissolution pattern of conventional formulation (Temodal ®). Preferably, the hydrophilic lubricants and hydrophobic lubricants may be contained at an amount of 0.05 to 3 parts by weight per 100 parts by weight of temozolomide.

The therapeutically effective amount of temozolomide may be contained in pharmaceutical composition and may be adjustable by various factors such as types and contents of active ingredients and other ingredients, formulation type, ages, weight, health condition, gender and diet of patients, administration time, administration route, secretion of the formulation, treatment period and drug co-administered. For example, the pharmaceutical formulation of an embodiment of the present invention may be administered at 75mg/m² to 200mg/m² per one day in single or multiple doses for an adult. However, it is obvious for a person skilled in the art that the active ingredient should not be contained in an excessive amount to cause side effects.

Preferably, the temozolomide may be contained at 3 to 60 wt% of the total weight of the pharmaceutical composition.

The pharmaceutical composition may be preferably provided as an oral formulation such as a capsule, a powder, or a tablet, but not is limited thereto.

Preferably, 90wt % or more, or substantially a total amount of temozolomide may be contained within the granules of the pharmaceutical composition.

Another example of the present invention provides a method of preparing granules described above and a pharmaceutical composition comprising the granules.

According to one preferable example of the present invention, the granule may be prepared by performing a) mixing temozolomide with tartaric acid as a pH stabilizer; and b) forming dry-granules with the mixture followed by sizing, and preferably, may be prepared by further comprising c) mixing the sized granules with one or more pharmaceutically acceptable additives.

Hereinafter, the methods of preparing granules described above and a pharmaceutical composition comprising the granules are described based on the preparation of the pharmaceutical composition.

The method of preparing the pharmaceutical composition with improved stability may comprise a) mixing temozolomide with tartaric acid as a pH stabilizer; b) forming dry-granules with the mixture and sizing the granules; and c) mixing the sized granules with one or more pharmaceutically acceptable additives.

The step a) may preferably include adding one or more pharmaceutically acceptable additives comprising anti-caking agents.

By adding anti-caking agents in addition to the active ingredients, temozolomide and a pH stabilizer, good flowability and compression molding property of powder can be obtained for lubricous property in the granulation, resulting in prevention of adhesion of active ingredients and the caking of temozolomide.

The step a) may further include adding one or more pharmaceutically acceptable additives generally used in the art such as excipients, binders, disintegrants, lubricants and diluents and conventionally used desiccants, sweetening agents or moisture absorbents, etc.

The mixture prepared by the step a) may be granulated by the step b).

The step b) may be a dry-granulation method for uniform stability of granules, and may more preferably produced using roller-compacting. A step of sizing or sieving the roller-compacted mixture with a fitz-mill or an oscillator may be further included to produce a proper size of granules.

The granules sized from step b) may further comprise one or more pharmaceutically acceptable additives in step c) to provide a pharmaceutical composition with improved stability.

The pharmaceutically acceptable additives of step c) refers to post-mixed additives as described above, and preferably includes sugars, starches, polysaccharide cellulose derivatives or mixture thereof. One person skilled in the art may properly choose one or more pharmaceutically acceptable additives from conventional additives without limitation.

Preferably, the pharmaceutically acceptable additives of the step c) may be a mixture of hydrophilic lubricants and hydrophobic lubricant in weight ratio of 2:1 to 1:5.

By mixing the hydrophilic lubricants and the hydrophobic lubricant with granules, the change in dissolution pattern caused by granulation process may be prevented, thereby providing the formulation having an equivalent dissolution pattern to conventional formulation, as well as excellent flowability and with remarkably improved stability.

Among pharmaceutically acceptable additives of step a), highly-hydrophilic additives, for example, lactose etc. may be mixed with the granules prepared in step b) by adding in step c) instead of step a), thereby improving the stability and maintaining equivalent dissolution pattern to the conventional formulation.

The pH stabilizers, anti-caking agents, pharmaceutically acceptable additives (additives in granules and post-mixed additives) and dry-granulation are the same as described above.

The pharmaceutical composition with improved stability obtained after step c) may be provided as various formulations such as a capsule or tablet.

Oral formulations such as capsule, powder, granule and tablet are preferable but are not limited thereto.

The temozolomide-containing oral formulation may be used for preventing or treating brain tumor such as glioma, glioblastoma multiforme and ependymoma as well as other proliferative diseases and may be useful as an oral formulation with improved stability in exterior circumstance such as pH and moisture.

### Brief Description of the Drawings

Fig. 1 shows property changes of the formulation prepared by simple-mixing method (Comparative Example 2: A) and the granulated formulation (Comparative Example 1: B) which were tested after 1 month from preparation according to Test Example 1.
Fig. 2 shows property changes of the dry-granulated formulation (Example 1), the wet-granulated formulation (Comparative Example 1) and the control drug (Comparative Example 3) which were tested after 1 month from preparation according to Test Example 1.
Fig. 3 shows a dissolution test result performed by Test Example 2.
Fig. 4 shows a stability test result performed by Test Example 3.
Fig. 5 shows a dissolution test result performed by Test Example 4.

### Examples

This invention will be better understood from the following preferable examples. However, the described examples are merely illustrative of, and are not intended to, nor should be intended to, limit the invention as described more fully in the claims which follow thereafter.

### [Components and Contents of Example 1 and Comparative Examples 1 to 3]

Capsules of Example 1 and Comparative Examples 1 to 3 were prepared according to the following components, contents and methods in Table 1.

**[Table 1]**

| Components (unit: wt%) | Example 1 | Comparative Example 1 | Comparative Example 2 | Comparative Example 3 |
|---|---|---|---|---|
| Temozolomide | 55.5% | 55.5% | 55.5% | Control drug: Temodal 260 mg capsule |
| Lactose | 34.3% | 34.3% | 34.3% | |
| Tartaric acid | 2.0% | 2.0% | 2.0% | |
| Silicon dioxide | 0.2% | 0.2% | 0.2% | |
| Sodium starch glycolate | 5.0% | 5.0% | 5.0% | |
| Stearic acid | 3.0% | 3.0% | 3.0% | |
| Total | 100% | 100% | 100% | |
| Method for Preparation | dry granulation | wet granulation | Simple-mixing | |

### Example 1: Dry-Granulation

After sieving and mixing 25g of temozolomide and 0.9g of tartaric acid, 15.4g of lactose, 0.07g of silicon dioxide and 2.25g of sodium starch glycolate were added and mixed, dry-granulated with a roller compactor(manufacturer: CHAMUNDA Pharma Machinery PVT. LTD, Model name: CPMMRC-100/25) and sized through a 30-mesh sieve. 1.35g of stearic acid was added and mixed for 5 minutes. The mixture was precisely measured to be 450mg and was filled to a 0-size capsule.

### Comparative Example 1: Wet-granulation

0.9g of tartaric acid was sieved and dissolved into 4ml of 99%(v/v) ethanol, wetted into a mixture of 25g of temozolomide, 15.4g of lactose and 0.07g of silicon dioxide for wet-granulation, dried in convection oven for 30 minutes and sized with a 30 mesh sieve. 1.35g of stearic acid was added and mixed for 5 minutes. 450 mg of the mixture was precisely taken and filled to a 0 size capsule.

### Comparative Example 2: Simple-Mixing

After mixing 25g of temozolomide and 0.9g of tartaric acid, 15.4g of lactose, 0.07g of silicon dioxide and 2.25g of sodium starch glycolate was added and sized through a 30 mesh sieve. 1.35g of stearic acid was added and mixed for 5 minutes. 450 mg of the mixture was precisely taken and filled to a 0 size capsule.

### Comparative Example 3

A commercially available Temodal 250mg capsule (Schering Corporation) was used as Comparative Example 3.

### <Test Example 1: Stability Test of temozolomide containing formulation>

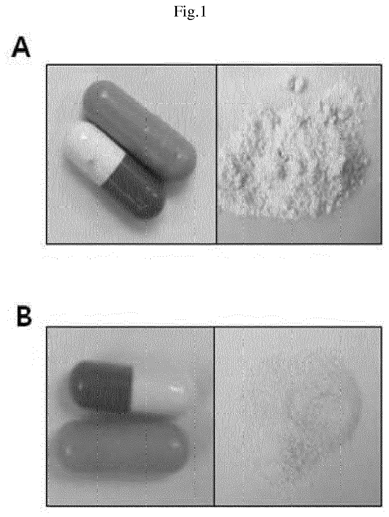

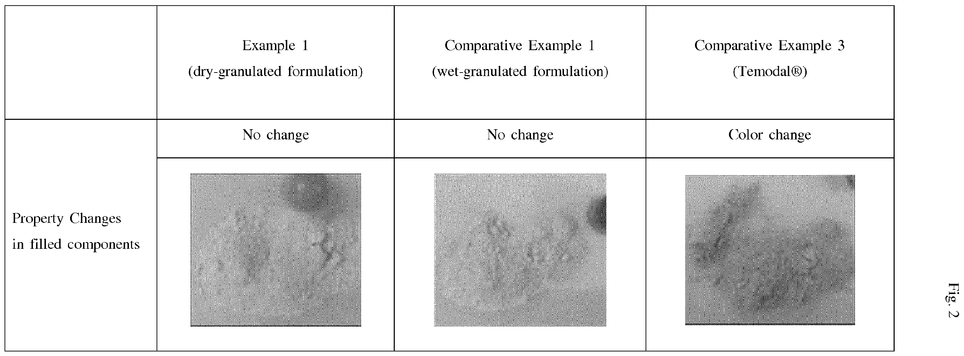

While the formulations of Examples 1 and Comparative Examples 1-3 were stored at 40°C and 75% RH, the changes in the property (water stability), content and related substances were analyzed with the lapse of time. The results are shown in Figs. 1 and 2 and Table 2. The related substances were analyzed with HPLC under the following condition.

### [HPLC analysis condition]

Column: length 150mm, diameter 4.6mm, 5 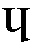 µm C18 column or equivalent columns
Detector: Ultraviolet Spectrophotometer (measuring wavelength 270nm)
Flow rate: 1.0mL/min
Injected amount: 10µl
Column temperature: 20∼30 °C
Mobile phase: solution obtained by mixing 960ml of 0.5% glacial acetic acid and 40ml of methanol, dissolving 0.94g/l (0.005M) of sodium hexanesulfonate and filtering with 0.45 µm filter.

As shown in Fig. 1, when checking the appearance changes after 1 month from the preparation, the formulation of the Comparative Example 2 prepared by simple mixing process without granulation (Fig1.A) showed changes in colors of the filled components in capsule and partial non-uniformity, compared to the granulated formulation of the Comparative Example 1(Fig1. B). The result showed that the formulation prepared by simple-mixing method has lower moisture-stability than the granulated formulation.

By comparing the formulations of Example 1 and Comparative Examples 1 and 3, after 3 months from the preparation, as shown in the Fig. 2, the formulations of Example 1 prepared by dry-granulation and Comparative Example 1 prepared by wet-granulation did not show large changes in properties of the filled components and the appearance of capsule and had outstanding moisture-stability compared to the Temodal capsule of Comparative Example 3.

As well as the changes in appearance, as a result of checking the changes in the content and related substances, while the content changed in the formulation of Comparative Example 3 was 3.5wt% after 1 month and 5.3 wt% after 2 months, the content change in the formulation of the Example 1 was only 2.9wt% after 2 months which is almost half of the formulation of Comparative Example 3. The related substances of the formulation of the Comparative Example 3 sharply increased to 0.13 wt% after 1 month, while the contents of related substances of the formulation of the Example 1 were only 0.02% after 2 months, which is only about 1/8 of the formulation of Comparative Example 3.

Compared to the formulation prepared by simple-mixing method in Comparative Example 2 and the commercially-available formulation of Comparative Example 3, the granulated formulation of Example 1 had excellent storage stability. Also, the dry-granulated formulation of Example 1 showed less change in appearance, content, and related substances than the wet-granulated formulation of the Comparative Example 1. The simple mixed formulation of Comparative Example 2 showed decrease in stability in a short time, and thus further experiment was not performed.

**[Table 2]**

| Components (unit: wt%) | Example 1 | Comparative Example 1 | Comparative Example 3 |
|---|---|---|---|
| Average content | 100.8 | 99.8 | 98.7 |
| Content change after 1 month | 99.6 | 98.62 | 95.2 |
| (40°C, 75% RH, bottle packaged) | | | |
| Content change after 2 months | 97.9 | 97.1 | 93.4 |
| (40°C, 75% RH, bottle packaged) | | | |
| Impurity amount after 1 month | 0.01 | 0.02 | 0.13 |
| (peak area of impurity/peak area of temozolomide) | | | |
| Impurity amount after 2 months | 0.02 | 0.02 | 0.16 |
| (peak area of impurity/peak area of temozolomide) | | | |

### Test Example 2: Property Comparison

The formulations of Examples and Comparative Examples were analyzed by dissolution test according to the test method for a temozolomide formulation described in US-FDA dissolution method. The result is shown in Fig. 2.

### Dissolution test (temozolomide capsule)

- Test method: USP type II(basket method)
- Eluent: 0.1N HCl 900mL
- Speed: 100rpm
- Dissolution time: 2hours

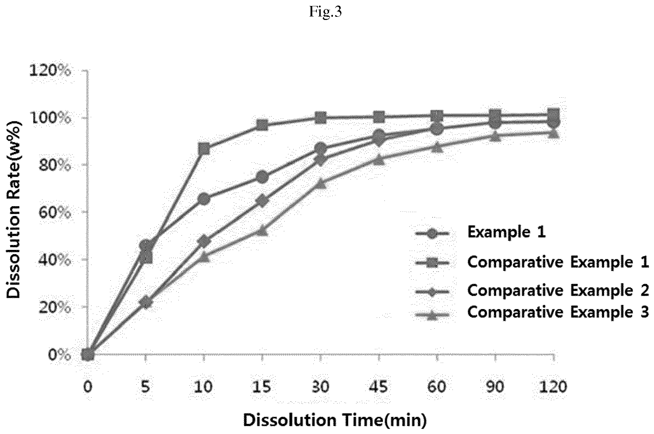

As shown in Fig. 3, according to the dissolution test result of Example 1 and Comparative Example 1, the granulated mixture of the active ingredient and the additives showed faster dissolution than the formulation of Comparative Example 2. The wet-granulated formulation of Comparative Example 1 had the highest dissolution rate, and the Temodal capsule of Comparative Example 3 showed the lowest rate. The dry-granulated formulation of Example 1 had dissolution rate lower than that of the formulation of the Comparative Example 1 and higher than that of the formulation of the Comparative Example 3. The simply mixed formulation of Comparative Example 2 showed the similar dissolution pattern to the control drug of Comparative Example 3.

The granulated formulation had shortened dissolution time, as it became larger and had a more uniform particle size than the powder and the components were mixed homogeneously. The wet-granulated formulation showed the most rapid dissolution pattern, because ethanol used as a solvent improved the solubility to the eluent. Accordingly, the test result confirmed that the dry-granulated formulation was more favorable than the wet-granulated formulation in the light of delaying dissolution.

### [Components and Content of the Examples 2 and 3]

To obtain more preferable properties of the dry-granulated formulation of Example 1, the dry-granule was prepared with different component, content and preparation method.

**[Table 3]**

| Components (unit: wt%) | Example 2 | Example 3 | Comparative Example 3 |
|---|---|---|---|
| Temozolomide | 55.5% | 55.5% | Control Temodal 250mg capsule |
| Lactose | 34.3% | 34.3% | |
| Tartaric acid | 2.0% | 2.0% | |
| Silicon dioxide | 0.2% | 0.2% | |
| Sodium starch glycolate | 5.0% | 5.0% | |
| Stearic acid | 3.0% | 1.7% | |
| Magnesium stearate | - | 1.3% | |
| Total | 100% | 100% | |
| Method | Dry granulation | Dry granulation | |

### Example 2

The dry-granules were prepared according to the same method as Example 1, except that lactose was added and mixed for 5 minutes after the dry-granulation and the sizing process, and was mixed for 5 minutes with the addition of 1.35g of stearic acid. 450mg of the mixture was taken precisely and was filled in a size-0 capsule.

### <Example 3>

The dry-granule was prepared according to the same method as Example 1, except that lactose was added and mixed for 5 minutes after the dry-granulation and the sizing process, and was mixed for 5 minutes with the addition of 0.68g of stearic acid and 0.57g of magnesium stearate. 449mg of the mixture was taken precisely and was filled in a size-0 capsule.

### <Test Example 3: Stability Test of Temozolomide Containing Formulation>

While the formulations of Examples 2 and 3 and Comparative Example 3 were stored at 40°C and 75% RH, the changes in the appearance, content and related substances were analyzed with the lapse of time.

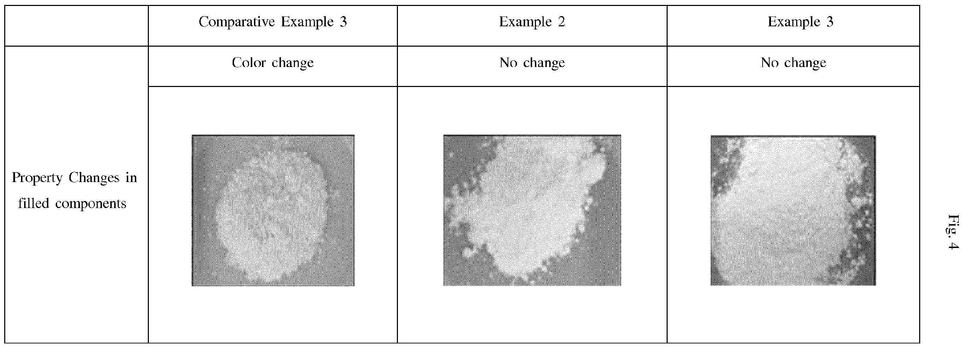

As shown in Fig. 4, the dry-granulated formulations of Examples 2 and 3 did not show large changes in appearance of the filled components and had outstanding water-stability compared to the Temodal capsule of Comparative Example 3.

As well as the changes in appearance, it was found that the changes in content and related substances did not show any significant differences.

As the content of related substances of the formulations of Examples 2 and 3 were significantly decreased, compare to that of Example 1, it was found that adding lactose after the dry-granulation process minimizes formation of the related substances.

**[Table 4]**

| Components (unit: wt %) | Example 2 | Example 3 |
|---|---|---|
| Average content | 98.9 | 99.3 |
| Content change after 3 month | 98.2 | 99.1 |
| (40°C, 75% RH, bottle packaged) | | |
| Related substance amount after 3 month | 0.007 | 0.006 |
| (peak area of related substance/peak area of temozolomide) | | |

### Test Example 4: Property Comparison

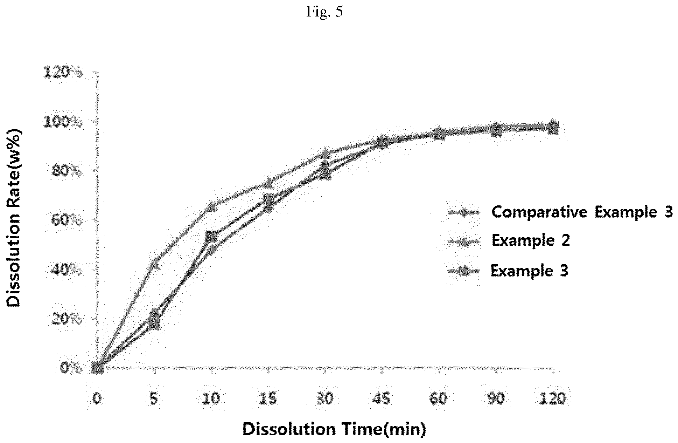

The formulations of the Examples 2 and 3 and the Comparative Example 3 were analyzed by dissolution test according to test method of Test Example 2. The result is shown in Fig. 5.

As shown in Fig. 5, the formulation of Example 2, which was prepared by mixing a highly hydrophilic excipient, lactose, as a extra-granular mixture together with a lubricant, stearic acid, after the dry-granulation and the sizing process, rather than granulating it together with the active ingredient, was found to have lower dissolution rate. However, the dissolution rate-lowering effect was less than that of formulation of Comparative Example 3. By mixing magnesium stearate, a highly hydrophobic lubricant, with the conventional stearic acid in a proper ratio (Example 3), it was found that it had almost the same dissolution pattern as the control drug.

As found from the above, the color and the capsule shape of the formulation stored in the accelerated condition have changed because the temozolomide formulation is unstable to pH and moisture, but the granulated formulations reduced the stability-decrease by mixing the drug and the stabilizer homogenously. Also, remarkably high stability was obtained compared to the control drug by selecting a type of the lubricant and its ratio to obtain a formulation showing similar property to the control drug.

## Claims

1. A capsule comprising a dry granule comprising temozolomide as an active ingredient and tartaric acid as a pH stabilizer.

2. The capsule of claim 1, wherein the pH stabilizer is in range of 1 to 50 parts by weight per 100 parts by weight of the temozolomide.

3. The capsule of claim 1, further comprising an anti-caking agent.

4. The capsule of claim 3, wherein the anti-caking agent is in range of 0.05 to 0.1 parts by weight per 100 parts by weight of the temozolomide.

5. The capsule of claim 1, further comprising one or more pharmaceutically acceptable additives.

6. The capsule of claim 5, wherein the pharmaceutically acceptable additive comprises a mixture of a hydrophilic lubricant and a hydrophobic lubricant.

7. The capsule of claim 6, wherein the hydrophilic lubricant is at least one selected from the group consisting of stearic acid, sodium lauryl sulfate, polyethylene glycol and mixtures thereof.

8. The capsule of claim 6, wherein the hydrophobic lubricant is at least one selected from the group consisting of magnesium stearate, talc, silicon dioxide, starch and mixtures thereof.

9. The capsule of claim 6, wherein the mixture of a hydrophilic lubricant and a hydrophobic lubricant contains the hydrophilic lubricant and the hydrophobic lubricant at a weight ratio of 2:1 to 1:5.

10. A method for preparing a capsule with improved stability comprising:
a) mixing temozolomide and tartaric acid as a pH stabilizer to produce a mixture;
b) forming dry granules from the mixture and sizing the dry granules; and
c) mixing the sized dry granules with one or more pharmaceutically acceptable additive.

11. The method of claim 10, wherein the step a) further comprises mixing one or more pharmaceutically acceptable additive comprising an anti-caking agent.

12. The method of claim 11, wherein the pharmaceutically acceptable additive of the step c) comprises a mixture of a hydrophilic lubricant and a hydrophobic lubricant at a weight ratio of 2:1 to 1:5.

13. The method of claim 11, wherein the hydrophilic excipient of the pharmaceutically acceptable additives is mixed at the step c) instead of the step a).

## Patentansprüche

1. Kapsel, die ein Trockengranulat aufweist, das Temozolomid als aktiven Bestandteil und Weinsäure als pH-Stabilisator aufweist.

2. Kapsel nach Anspruch 1, wobei der pH-Stabilisator im Bereich von 1 bis 50 Gewichtsteilen pro 100 g Gewichtsteile Temozolomid liegt.

3. Kapsel nach Anspruch 1, die weiterhin ein Trennmittel aufweist.

4. Kapsel nach Anspruch 3, wobei das Trennmittel im Bereich von 0,05 bis 0,1 Gewichtsteile pro 100 g Gewichtsteile Temozolomid liegt.

5. Kapsel nach Anspruch 1, die weiterhin einen oder mehrere pharmazeutisch annehmbare Zusatzstoff(e) aufweist.

6. Kapsel nach Anspruch 5, wobei der pharmazeutisch annehmbare Zusatzstoff eine Mischung aus einem hydrophilen Gleitmittel und einem hydrophoben Gleitmittel aufweist.

7. Kapsel nach Anspruch 6, wobei das hydrophile Gleitmittel wenigstens eines ist, das ausgewählt ist aus der Gruppe bestehend aus Stearinsäure, Natriumlaurylsulfat, Polyethylenglykol und Mischungen davon.

8. Kapsel nach Anspruch 6, wobei das hydrophobe Gleitmittel wenigstens eines ist, das ausgewählt ist aus der Gruppe bestehend aus Magnesiumstearat, Talk, Siliziumdioxid, Stärke und Mischungen davon.

9. Kapsel nach Anspruch 6, wobei die Mischung eines hydrophilen Gleitmittels und eines hydrophoben Gleitmittels das hydrophile Gleitmittel und das hydrophobe Gleitmittel in einem Gewichtsverhältnis von 2:1 bis 1:5 enthält.

10. Verfahren zum Herstellen einer Kapsel mit verbesserter Stabilität, das aufweist:
a) Mischen von Temozolomid und Weinsäure als pH-Stabilisator, um eine Mischung herzustellen;
b) Ausbilden von Trockengranulaten aus der Mischung und größenmäßiges Anpassen der Trockengranulate; und
c) Mischen der in der Größe angepassten Trockengranulate mit einem oder mehreren pharmazeutisch annehmbaren Zusatzstoff(en).

11. Verfahren nach Anspruch 10, wobei der Schritt a) weiterhin das Mischen von einem oder mehreren pharmazeutisch annehmbaren Zusatzstoffen aufweist, die ein Trennmittel aufweisen.

12. Verfahren nach Anspruch 11, wobei der pharmazeutisch annehmbare Zusatzstoff von Schritt c) eine Mischung aus einem hydrophilen Gleitmittel und einem hydrophoben Gleitmittel in einem Gewichtsverhältnis von 2:1 bis 1:5 aufweist.

13. Verfahren nach Anspruch 11, wobei der hydrophile Hilfsstoff der pharmazeutisch annehmbaren Zusatzstoffe in Schritt c) anstelle von Schritt a) gemischt wird.

## Revendications

1. Capsule comprenant un granule sec comprenant du témozolomide en tant qu'ingrédient actif et de l'acide tartrique en tant que stabilisateur du pH.

2. Capsule selon la revendication 1, dans laquelle le stabilisateur du pH se trouve dans la plage de 1 à 50 parties en poids pour 100 parties en poids du témozolomide.

3. Capsule selon la revendication 1, comprenant en outre un agent antiagglomérant.

4. Capsule selon la revendication 3, dans laquelle l'agent antiagglomérant est dans la plage de 0,05 à 0,1 partie en poids pour 100 parties en poids du témozolomide.

5. Capsule selon la revendication 1, comprenant en outre un ou plusieurs additifs pharmaceutiquement efficaces.

6. Capsule selon la revendication 5, dans laquelle l'additif pharmaceutiquement acceptable comprend un mélange d'un lubrifiant hydrophile et d'un lubrifiant hydrophobe.

7. Capsule selon la revendication 6, dans laquelle le lubrifiant hydrophile est au moins un sélectionné dans le groupe constitué par l'acide stéarique, le lauryl sulfate de sodium, le polyéthylène glycol et des mélanges de ceux-ci.

8. Capsule selon la revendication 6, dans laquelle le lubrifiant hydrophobe est au moins un sélectionné dans le groupe constitué par le stéarate de magnésium, le talc, le dioxyde de silicium, l'amidon et des mélanges de ceux-ci.

9. Capsule selon la revendication 6, dans lequel le mélange d'un lubrifiant hydrophile et d'un lubrifiant hydrophobe contient le lubrifiant hydrophile et le lubrifiant hydrophobe en un rapport en poids de 2/1 à 1/5.

10. Procédé de préparation d'une capsule ayant une stabilité améliorée comprenant :
a) le mélange de témozolomide et d'acide tartrique en tant que stabilisateur de pH pour produire un mélange ;
b) la formation de granules à partir du mélange et le dimensionnement des granules secs ; et
c) le mélange des granules secs dimensionnés avec un ou plusieurs additifs pharmaceutiquement acceptables.

11. Procédé selon la revendication 10, dans lequel l'étape a) comprend en outre le mélange d'un ou plusieurs additifs pharmaceutiquement acceptables comprenant un agent antiagglomérant.

12. Procédé selon la revendication 11, dans lequel l'additif pharmaceutiquement acceptable de l'étape c) comprend un mélange d'un lubrifiant hydrophile et d'un lubrifiant hydrophobe en un rapport en poids de 2/1 à 1/5.

13. Procédé selon la revendication 11, dans lequel l'excipient hydrophile des additifs pharmaceutiquement acceptables est mélangé à l'étape c) plutôt qu'à l'étape a).
